# EUROPEAN PATENT SPECIFICATION

(11) **EP 1 803 386 B1**
(45) Date of publication and mention of the grant of the patent: **04.06.2014**
(21) Application number: 06124700.3
(22) Date of filing: 23.11.2006
(51) Int. Cl.: A61B 1/06, G02B 23/24, H04N 5/235

(54) **Device for in-vivo illumination**
Vorrichtung für Beleuchtung in vivo
Dispositif d'éclairage in vivo

(30) Priority: 29.12.2005 US 319771
(43) Date of publication of application: 04.07.2007
(73) Proprietor: Given Imaging Ltd., 20692 Yoqneam (IL)
(72) Inventor: Pascal, Amit, 34980, Haifa (IL)
(74) Representative: Vaughan, Christopher Tammo

(56) References cited:
- AU-A1- 2004 201 752
- GB-A- 2 381 305
- US-A- 4 986 262
- US-A1- 2003 223 248
- US-A1- 2005 148 842
- US-B1- 6 240 312

## Description

### FIELD OF THE INVENTION

The present invention relates to a device for in-vivo imaging, more specifically to a device for providing illumination in-vivo

### BACKGROUND OF THE INVENTION

In-vivo imaging devices, such as swallowable capsules or other devices may move through a body lumen, imaging as they move along. In an in-vivo imaging device having a certain field of view (FOV) and incorporating an illumination system, the illumination is achieved by a light source(s) having a certain field of illumination (FOI).

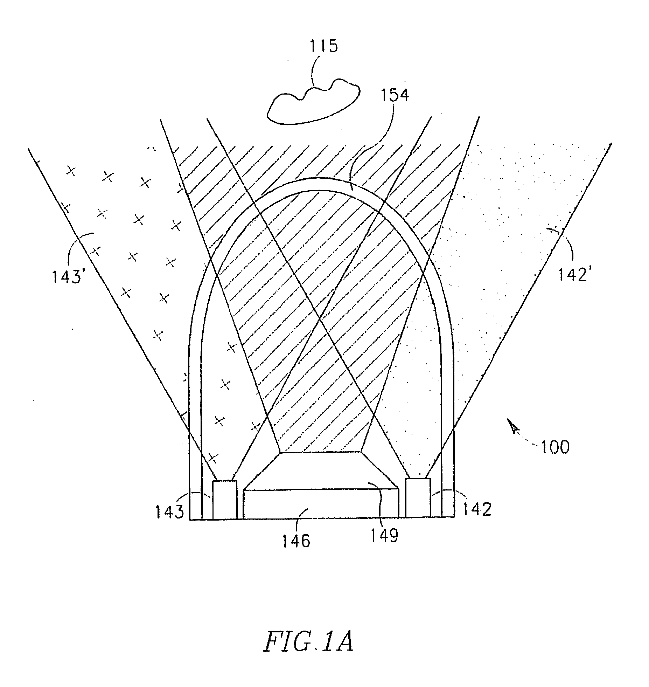

Reference is now made to Fig. 1A, showing a schematic two dimensional presentation of an optical system according to an embodiment of the prior art. Referring to Fig. 1A, optical system generally referenced as 100 may be included in, an in-vivo imaging device, but may be included in other suitable devices, such as an endoscope, trocar, or other in-vivo imaging device. Optical system 100 may include, for example, light sources 142 and 143, an imager 146, and one or more lenses 149 disposed behind a viewing window such as optical dome 154, for viewing, for example, a target or object 115. One, two, or more than two illumination sources may be used. FOI 142' (indicated by dots) defines the area illuminated by light source 142, while FOI 143' (indicated by crosses) defines the area illuminated by light source 143.

The FOI illuminated by each light source, such as light sources 142 and 143, is typically stretched over a relatively wide area, with a varying intensity of illumination that is proportional to the inverse square of distance from the light source.

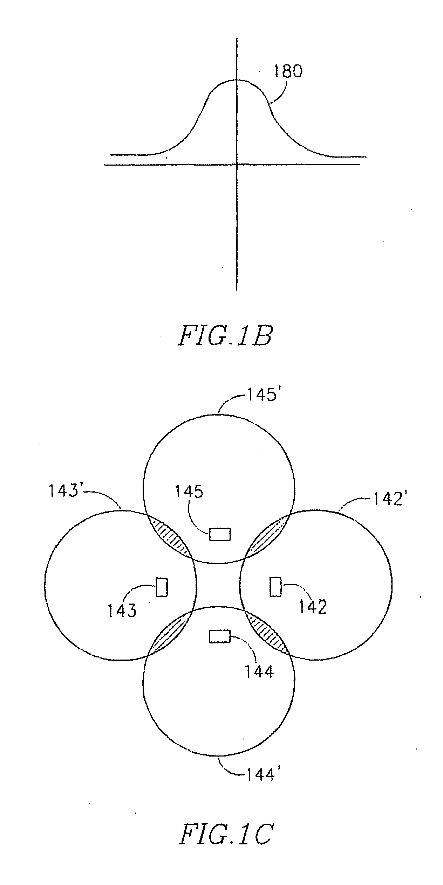

Fig. 1B is an exemplary graphical illustration of the illumination distribution within a FOI, such as FOI 142' or 143', of a single light source, for example a commercially available white LED. The illumination distribution within a FOI of a light source is best described as a Gaussian distribution as characterized by Gaussian curve 180. In cases where four light sources are employed, for example within an optical system of an in-vivo imaging device, four overlapping areas are created between the FOI of each light source. For example, as depicted in Fig. 1C, for each light source 142, 143, 144 and 145 four FOI 142', 143', 144' and 145' exist, respectively. The partial overlaps between FOI of each light source may create four distinct areas which are strongly illuminated whereas in other areas illumination may be diminished in comparison. For example, the areas created at the conjunction of the four overlapping FOI 142', 143', 144' and 145 ' (the four shaded areas) are strongly illuminated, while other areas are more weakly illuminated.

There is a need for an in vivo device that will provide unvarying, uniform illumination in the in-vivo device field of view.

It is known from US 2005/148842 to provide a wireless capsule including a micro-spectrometer and/or imaging system including a light source. The light source may one or more color LEDs that can be combined using a hologram to form a wideband light source with a controlled spectral intensity distribution.

### SUMMARY OF THE INVENTION

There is provided, in accordance with some embodiments of the present invention an in-vivo imaging device having an illumination unit which may provide uniform illumination as set forth in the accompanying claims. According to one embodiment of the present invention the illumination unit may include, for example, a base or support for holding one or more illumination units. The illumination unit comprises at least two light sources, such as light emitting diodes (LEDs) or Organic LEDs (OLEDs) or other suitable illumination sources. The beam shaping units may homogenize and beam shape the light source output for a given field of view and a given depth of view of the in-vivo imaging device.

### BRIEF DESCRIPTION OF THE DRAWINGS

The principles and operation of the apparatus according to the present invention may be better understood with reference to the drawings, and the following description, it being understood that these drawings are given for illustrative purposes only and are not meant to be limiting, wherein:
Fig. 1A shows a schematic illustration of an optical system according to one embodiment of the prior art;
Fig. 1B is an exemplary graphical illustration of an illumination distribution of a light source, according to one embodiment of the prior art;
Fig. 1C shows a schematic illustration of a field of illumination, according to one embodiment of the prior art;
Fig. 2 is a schematic illustration of an in vivo imaging device, according to an embodiment of the present invention;
Figs. 3A-3B are schematic illustrations of an illumination unit, according to embodiments of the present invention;
Fig. 3C is a graphical representation of an angular luminance distribution;
Fig. 4A is a schematic illustration of an optical system;
Fig. 4B is a graphical representation of an angular luminance distribution;
Fig. 4C is a graphical representation of an angular luminance distribution according to yet another embodiment of the present invention; and
Fig. 5 is a flowchart depicting a method for producing an illumination unit, according to an embodiment of the present invention.

It should be noted that for simplicity and clarity of illustration, elements shown in the figures have not necessarily been drawn to scale. For example, the dimensions of some of the elements may be exaggerated relative to other elements for clarity. Furthermore, where considered appropriate, reference numerals may be repeated among the figures to indicate corresponding or analogous elements throughout the serial views.

### DETAILED DESCRIPTION OF THE INVENTION

The following description is presented to enable one of ordinary skill in the art to make and use the invention as provided in the context of a particular application and its requirements. Various modifications to the described embodiments will be apparent to those with skill in the art, and the general principles defined herein may be applied to other embodiments. Therefore, the present invention is not intended to be limited to the particular embodiments shown and described, but is to be accorded the widest scope consistent with the principles and novel features herein disclosed. In the following detailed description, numerous specific details are set forth in order to provide a thorough understanding of the present invention. However, it will be understood by those skilled in the art that the present invention may be practiced without these specific details. In other instances, well-known methods, procedures, and components have not been described in detail so as not to obscure the present invention.

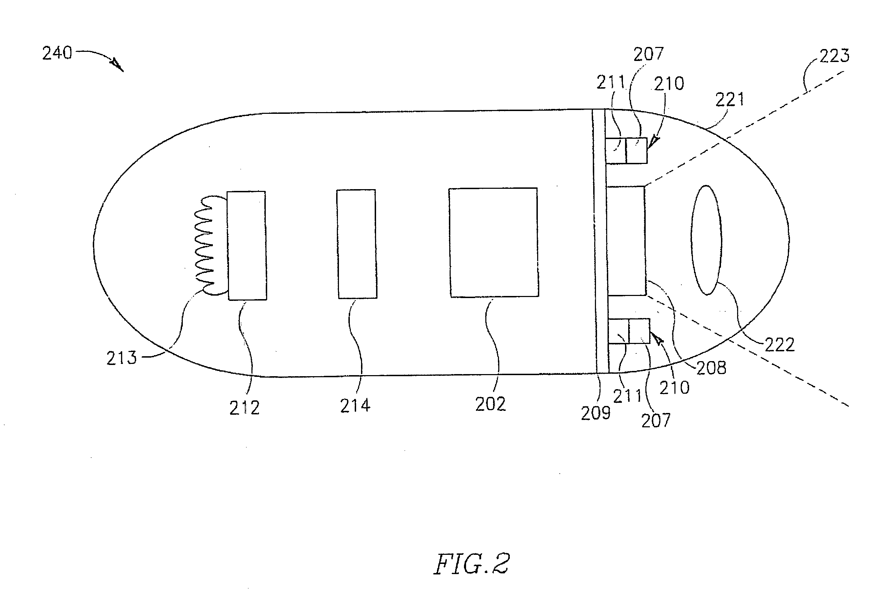

Reference is now made to Fig. 2, which schematically illustrates an in vivo imaging device according to an embodiment of the invention. According to one embodiment the device 240 may include a housing 209 and a dome or viewing window 221. The housing 209 may contain an imaging system for obtaining images from inside a body lumen, such as the GI tract. The imaging system may include one or more illumination units 210, an image sensor for example an imager 208 and an optical unit 222 which focuses the images onto the imager 208 The illumination unit 210 may illuminate the inner portions of the body lumen through viewing window 221. According to some embodiments of the present invention the illumination unit 210 may include a light source 211, such as a white LED and/or an OLED, and an optical unit such as a beam shaping unit 207 to ensure even distribution of the light in the field of view 223 of device 240, and, according to some embodiments, to enable the use of only one light source in the device 240. Device 240 may further include a control unit 214, a transmitter 212 a power source 202, such as a silver oxide battery, that provides power to the electrical elements of the device 240, and an antenna 213 for transmitting and/or receiving signals. For example, an antenna 213 may be used to transmit image signals from the imager 208. A suitable imager 208 may be, for example, a "camera on a chip" type CMOS imager. Other suitable types of imagers may be used, for example, a CCD imager. The single chip camera can provide signals for either black and white or color images. A suitable transmitter may comprise a modulator which receives the image signal (either digital or analog) from the CMOS imaging camera, a Radio Frequency (RF) amplifier, an impedance matcher and an antenna. A processor, e.g., for processing the image data may be included in the device. The processor or processing circuitry may be integrated in the sensor or in the transmitter

According to some embodiments the device 240 may be capsule shaped and can operate as an autonomous endoscope for imaging the Gl tract. However, other devices, such as devices designed to be incorporated in an endoscope, catheter, stent, needle, etc., may also be used, according to embodiments of the invention. Furthermore, the device 240 need not include all the elements described above. For example, the device 240 need not include an internal power source; power may be provided from an external source, for example, as known in the art.

According to one embodiment of the invention, various components of the device 240 may be disposed on a support 209 such as a circuit board including for example rigid and flexible portions; preferably the components are arranged in a stacked vertical fashion in alternate embodiments, other arrangements of components may be placed on a circuit board having rigid portions connected by flexible portions. Such circuit boards may be similar to embodiments described in US Patent Application number 2006/0004257 entitled IN VIVO DEVICE WITH FLEXIBLE CIRCUIT BOARD AND METHOD FOR ASSEMBLY THEREOF, and US Patent Application number 2004/0171914 entitled IN VIVO SENSING DEVICE WITH A CIRCUIT BOARD HAVING RIGID SECTIONS AND FLEXIBLE SECTIONS. In alternate embodiments, a circuit board having rigid portions and flexible portions may be used to arrange and hold components in other in vivo sensing devices, such as a swallowable capsule measuring pH, temperature or pressure, or in a swallowable imaging capsule having components other than those described above.

Device 240 typically may be or may include an autonomous swallowable capsule, but device 240 may have other shapes and need not be swallowable or autonomous Embodiments of device 240 are typically autonomous, and are typically self-contained, For example, device 240 may be a capsule or other unit where all the components are substantially contained within a container or shell, and where device 240 does not require any wires or cables to, for example, receive power from an external source or transmit information. Device 240 may communicate with an external receiving and display system to provide display of data, control, or other functions. Other embodiments may have other configurations and capabilities. For example, components may be distributed over multiple sites or units. Control information may be received from an external source.

Devices according to embodiments of the present invention, including imaging, receiving, processing, storage and/or display units suitable for use with embodiments of the present invention, may be similar to embodiments described in U.S. Patent No. 5,604,531 to Iddan et al., entitled IN VIVO VIDEO CAMERA SYSTEM and/or US Patent No. 7,009,634 entitled A DEVICE AND SYSTEM FOR IN VIVO IMAGING, both of which are assigned to the common assignee of the present invention. Of course, devices and systems as described herein may have other configurations and other sets of components.

In one embodiment, all of the components may be sealed within the device body (the body or shell may include more than one piece); for example, a control unit 214, an imager 208, an illumination unit 210, power source 202, and transmitting 212 and control 214 units, may all be sealed within the device body,

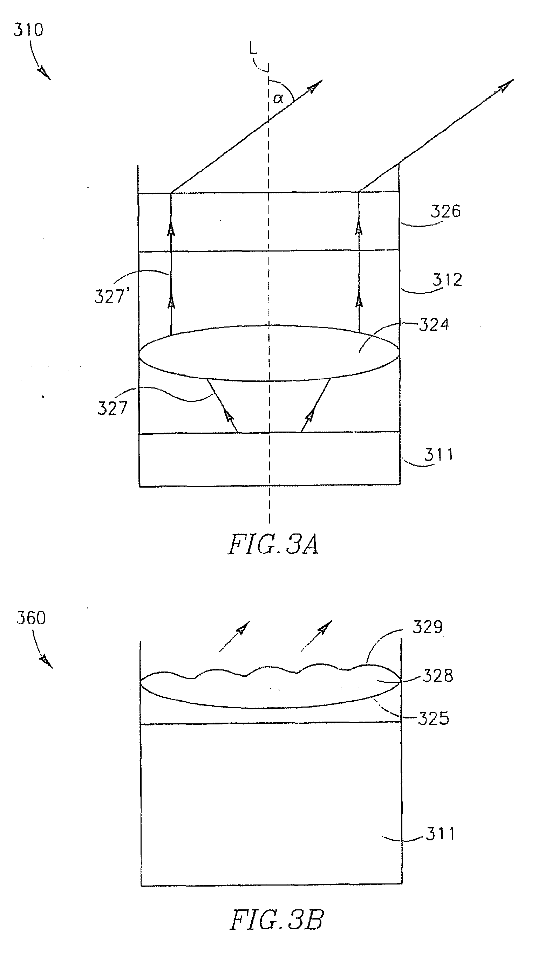

Reference is now made to Fig. 3A showing a schematic closer view from the side of an illumination unit 310, in accordance with one embodiment of the present invention. According to some embodiments the illumination unit 310, may include a light source 311 such as an LED (monochromatic or white) or an OLED, and a beam shaping unit e.g. a micro optical unit 312 for homogenizing and/or beam shaping the light source 311 output. According to one embodiment the micro optical unit 312 is positioned in close proximity to the light source 31 and may include, for example a refractive element such as a lens 324 and a diffractive optical element (DOE) 326. The objective of the lens 324 is to funnel and shape the light beam emitted from the light source 311 so that the light beam will run parallel (in relation to a longitudinal axis L of the illumination unit 310) before it hits the DOE 326. For example, a light beam emitted from light source 311 e.g. a divergent light beam 327, hits lens 324, bends and become, for example a collimated light beam 327'. The re-directed light beam, such as the collimated light beam 327' may hit DOE 326 and may be shifted at an angle α (in relation to a longitudinal axis L of the illumination unit 310).

Fig. 3B illustrates an illumination unit 360 according to another embodiment of the present invention. According to some embodiments, the illumination unit 360, may include a light source 311 such as a white or monochromatic light source, such as an LED or an OLED, and a beam shaping unit e.g. a micro optical element such as a lens 328. The lens 328 may include different surfaces on each side. For example the lens 328 may include a refractive surface 325 on the lens side facing the light source 311 and DOE surface 329 on the opposite side. The refractive surface 325 may be used for breaking and re-directing the light beam emitted from the light source 311. Thus, a light beam emitted from light source 311 hitting the refractive surface 325 will turn from a divergent beam to a collimated beam. DOE surface 329 may be used for homogenizing and beam shaping the collimated light beam.

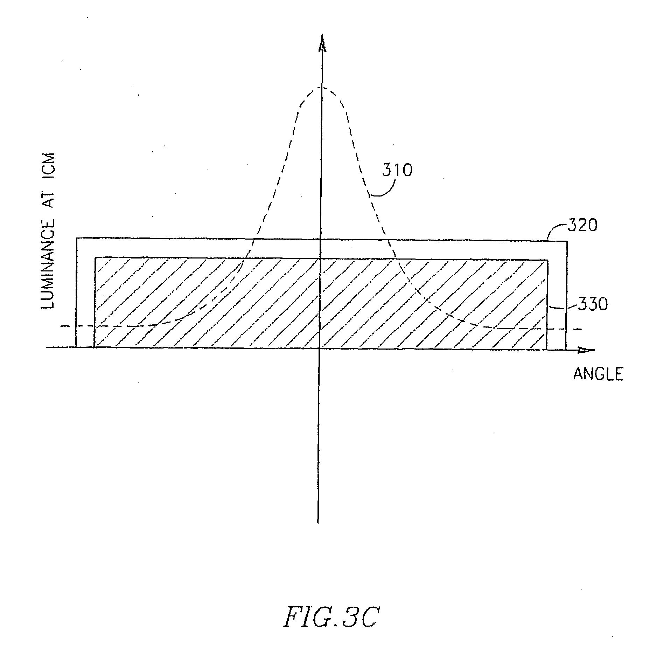

Fig 3C depicts a graphic representation of an angular luminance distribution of two different illumination units. Curve 310 (indicated by a segmented curve) depicts a Gaussian luminance distribution of an illumination unit that does not include a light beam shaping element, while curve 320 depicts a 'top hat' luminance distribution of a single illumination unit such as the illumination unit 310 (shown in Fig. 3A) that includes a light beam shaping element. The light beam shaping unit may convert the luminance characteristics of a single light source, for example from a Gaussian illumination distribution into a 'top hat' illumination distribution with an FWHM (Full-Width of Half Maximum) of about 110° - 160°. As can be seen, the light beam shaping unit is used to provide a high intensity focused illumination field that has a uniform appearance across the entire near FOV 330 of the imaging device, for example between 0-4cm from the in-vivo imaging device viewing window.

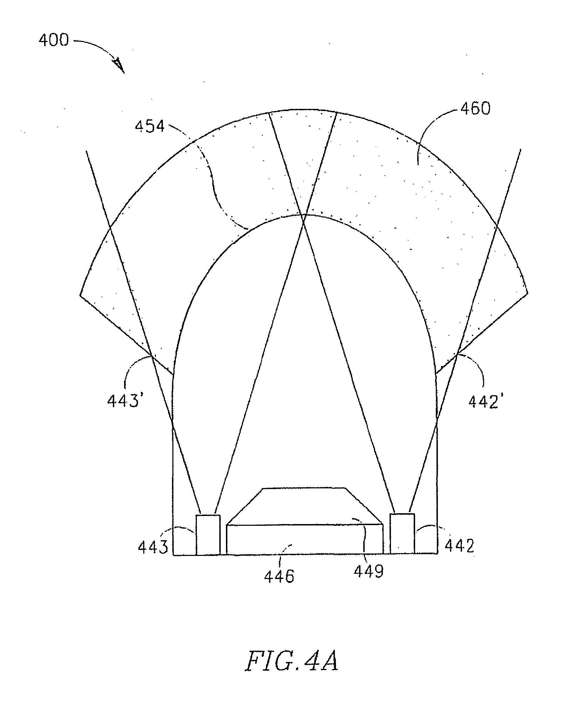

Reference is now made to Fig. 4A, showing a schematic two dimensional presentation of an optical system. Referring to Fig. 4A, an optical system generally referenced as 400 may be included in an in-vivo imaging device, but may be included in other suitable devices, such as an endoscope, trocar, or other in-vivo imaging devices Optical system 400 may include an imager 446, and one or more lenses 449 disposed behind a viewing window 454. The optical system 400 may include, for example only two illumination units such as illumination units 442 and 443. Alternatively, more illumination units may be included. The illumination units 442 and 443 may be similar to the illumination unit 310 shown in Fig. 3A. The illumination units 442 and 443 may be used for illuminating a FOV e.g. the near field of view 460 (indicated by dots) located, for example in the range of 0-5cm from the optical window 454 of the optical system 400. As shown in Fig. 4A, each illumination unit 442 and 443 may produce a high intensity focused light beam 442' and 443'.

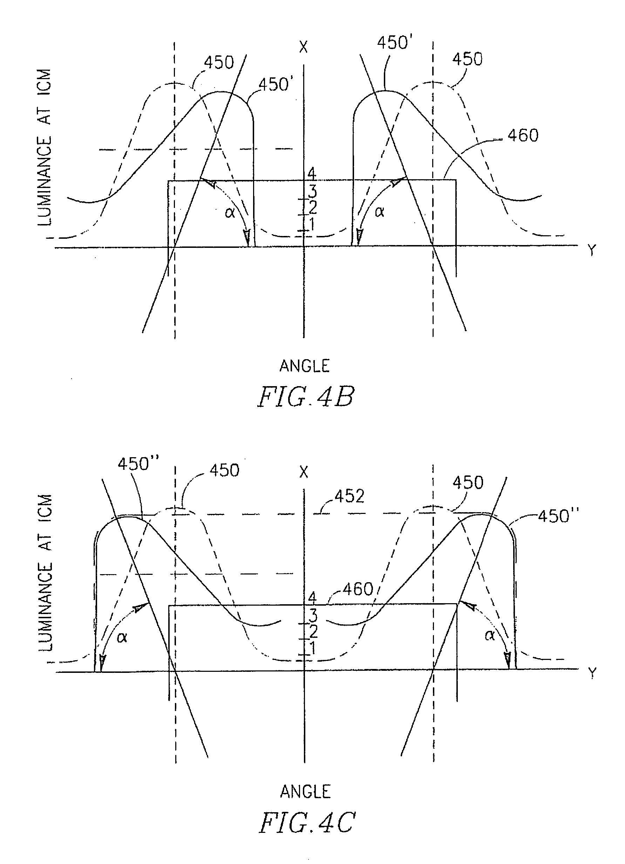

Fig. 4B depicts a graphic representation of an angular luminance distribution of two illumination units, such as the two illumination units 442 and 443 shown in Fig. 4A. Each of the two curves 450 (indicated by a segmented line) depicts a Gaussian luminance distribution of an illumination unit, such as the light source 142 shown in Fig 1A, which do not include a light beam shaping element, while each of the two curves 450' depicts a Gaussian luminance distribution of an illumination unit such as the illumination unit 310 (shown in Fig 3A) which includes a light beam shaping element.

As shown in Fig. 4B, by placing a light beam shaping element on each light source, such as light source 142 the peak of each Gaussian luminance distribution curve 450' may be biased towards the direction of axis X and forming an angle α with the axis Y, so that the two Gaussian luminance distribution curves 450' converge towards each other.

In accordance with another embodiment, Fig. 4C depicts a graphic representation of an angular luminance distribution of two illumination units, such as the two illumination units 442 and 443 shown in Fig. 4A. Each of the two curves 450 (indicated by a segmented line) depicts a Gaussian luminance distribution of an illumination unit, such as the light source 142 shown in Fig 1A, which do not include a light beam shaping element, while each of the two curves 450" depicts a Gaussian luminance distribution of an illumination unit such as the illumination unit 310 (shown in Fig. 3A) which includes a light beam shaping element.

As shown in Fig. 4C, by placing a light beam shaping element on each light source, such as light source 142 the peak of each Gaussian luminance distribution curve 450" may be biased away from the direction of axis X and forming an angle α with the axis Y, so that the two Gaussian luminance distribution curves 450" diverge away from each other. Thereby, the resultant combined luminance distribution of the light 452 (shown by the dash-dot curve) of the two Gaussian luminance distribution curves 450" that diverge away from each other is uniform. In accordance with some embodiments, the resultant distribution of the light 452 can be made uniform in the FOV of the in-vivo device, e.g. in the near field of view 460 located for example in the range of 0-5cm from the optical window of an in-vivo imaging device

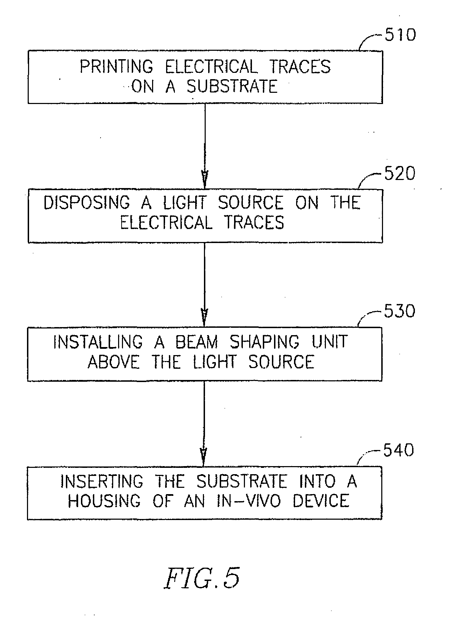

A method for producing an in vivo imaging device, which may include an illumination unit such as the illumination unit 210, according to different embodiments of the present invention is depicted in Fig. 5. According to some embodiments of the present invention, step 510 may include printing electrical traces on a substrate, such as a Printed Circuit Board (PCB). Step 520 may include disposing a light source, for example a white LED on the electrical traces. Step 530 may include installing a beam shaping unit above the light source, this step may include for example installing a refractive optical element above the light source and diffractive optical element above the refractive optical element. Step 540 may include inserting the substrate into a housing of an in vivo device. According to some embodiments the method may include providing an imager, typically by positioning the imager on the substrate. According to some embodiments other components of a swallowable imaging capsule may be provided, such as a transmitter, control unit and power source.

The foregoing description of the embodiments of the invention has been presented for the purposes of illustration and description. It is not intended to be exhaustive or to limit the invention to the precise form disclosed. It should be appreciated by persons skilled in the art that many modifications, variations, substitutions, changes, and equivalents are possible in light of the above teaching. It is, therefore, to be understood that the appended claims are intended to cover all such modifications and changes as fall within the scope of the invention as claimed.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. A device (240) for in vivo imaging comprising an illumination unit (210) having a longitudinal axis (X), said illumination unit (210) comprising at least two light sources (211), each of said at least two light sources (210) comprising a Gaussian luminance distribution, and each light source being provided with a beam shaping unit (207), wherein the beam shaping unit (207) is to adjust a beam direction of each light source (211) such that the peak of each Gaussian luminance distribution curve is angled to the longitudinal axis (X) such that the resultant combined luminance distribution of light is uniform in a field of view (223) of the device (240).

2. The device (240) according to claim 1, comprising a housing (290) and a viewing window (221), wherein the illumination unit (210) is contained within the housing and behind the viewing window.

3. The device (240) according to claim 1 or 2, wherein said beam shaping unit (207) comprises a refractive optical element.

4. The device (240) according to claim 1 or 2, wherein said beam shaping unit (207) comprises a diffractive optical element.

5. The device (240) according to claim 1 or 2, wherein said beam shaping unit (207) comprises an optical element said optical element including a refractive surface and a diffractive surface.

6. The device (240) according to any preceding claim, wherein said light source (211) is selected from the group consisting of an LED and an OLED.

7. The device (240) according to any preceding claim, wherein the illumination unit (210) is positioned on a support (209).

8. The device (240) according to any preceding claim, comprising an imager (208).

9. The device (240) according to any preceding claim, comprising a power source (202).

10. The device (240) according to any preceding claim, comprising a transmitter (212).

11. The device (240) according to any preceding claim, wherein said in-vivo imaging device (240) is an autonomous capsule.

12. The device (240) according to any preceding claim, wherein the field of view (223) is in the near field of view, in the range of 0 to 5cm from an optical window (221) of the device.

## Patentansprüche

1. Vorrichtung (240) zur in-vivo-Bildgebung, welche eine Beleuchtungseinheit (210) mit einer Längsachse (X) umfasst, wobei die Beleuchtungseinheit (210) mindestens zwei Lichtquellen (211) umfasst, wobei jede der mindestens zwei Lichtquellen (211) eine Gaußsche Luminanzverteilung umfasst, und wobei jede Lichtquelle mit einer Strahlenformereinheit (207) versehen ist, wobei die Strahlenformereinheit (207) dazu dient, die Strahlrichtung jeder Lichtquelle (211) anzupassen, so dass die Spitze jeder Gaußschen Luminanzverteilungskurve in einem Winkel zu der Längsachse (X) angeordnet ist, so dass die resultierende kombinierte Luminanzverteilung des Lichts in einem Sichtfeld (223) der Vorrichtung (240) einheitlich ist

2. Vorrichtung (240) nach Anspruch 1, mit einem Gehäuse (290) und einem Sichtfenster (221), wobei sich die Beleuchtungseinheit (210) in dem Gehäuse und hinter dem Sichtfenster befindet.

3. Vorrichtung (240) nach Anspruch 1 oder 2, wobei die Strahlenformereinheit (207) ein lichtbrechendes optisches Element umfasst.

4. Vorrichtung (240) nach Anspruch 1 oder 2, wobei die Strahlenformereinheit (207) ein beugendes optisches Element umfasst.

5. Vorrichtung (240) nach Anspruch 1 oder 2, wobei die Strahlenformereinheit (207) ein optisches Element umfasst, wobei das optische Element eine lichtbrechende Oberfläche und eine beugende Oberfläche aufweist.

6. Vorrichtung (240) nach einem der vorstehenden Ansprüche, wobei die Lichtquelle (211) aus der Gruppe ausgewählt wird, die eine LED und eine OLED umfasst.

7. Vorrichtung (240) nach einem der vorstehenden Ansprüche, wobei die Beleuchtungseinheit (210) auf einem Träger (209) positioniert ist.

8. Vorrichtung (240) nach einem der vorstehenden Ansprüche, wobei diese eine Bildgebungsvorrichtung (208) umfasst.

9. Vorrichtung (240) nach einem der vorstehenden Ansprüche, wobei diese eine Energiequelle (202) umfasst.

10. Vorrichtung (240) nach einem der vorstehenden Ansprüche, wobei diese einen Sender (212) umfasst.

11. Vorrichtung (240) nach einem der vorstehenden Ansprüche, wobei es sich bei der in-vivo-Bildgebungsvorrichtung (240) um eine autonome Kapsel handelt.

12. Vorrichtung (240) nach einem der vorstehenden Ansprüche, wobei sich dass Sichtfeld (223) in dem nahen Sichtfeld befindet, im Bereich von 0 bis 5 cm von einem optischen Fenster (221) der Vorrichtung.

## Revendications

1. Dispositif (240) pour l'imagerie in vivo, comprenant une unité d'éclairage (210) ayant un axe longitudinal (X), ladite unité d'éclairage (210) comprenant au moins deux sources lumineuses (211), chacune desdites au moins deux sources lumineuses (210) comprenant une distribution de luminance de Gauss, et chaque source lumineuse comprenant une unité de mise en forme de faisceau (207), dans lequel l'unité de mise en forme de faisceau (207) est conçue pour ajuster une direction de faisceau de chaque source lumineuse (211) de sorte que le pic de chaque courbe de répartition de la luminance de Gauss est incliné vers l'axe longitudinal (X) de sorte que la répartition de la luminance combinée résultante de lumière est uniforme dans un champ de vision (223) du dispositif (240).

2. Dispositif (240) selon la revendication 1, comprenant un boîtier (290) et un hublot (221), dans lequel l'unité d'éclairage (210) est contenue à l'intérieur du boîtier et derrière le hublot.

3. Dispositif (240) selon la revendication 1 ou 2, dans lequel ladite unité de mise en forme de faisceau (207) comprend un élément optique de réfraction.

4. Dispositif (240) selon la revendication 1 ou 2, dans lequel ladite unité de mise en forme de faisceau (207) comprend un élément optique de diffraction.

5. Dispositif (240) selon la revendication 1 ou 2, dans lequel ladite unité de mise en forme de faisceau (207) comprend un élément optique dudit élément optique comprenant une surface de réfraction et une surface de diffraction.

6. Dispositif (240) selon l'une quelconque des revendications précédentes, dans lequel ladite source lumineuse (211) est choisie dans le groupe constitué d'une LED et d'un écran OLED.

7. Dispositif (240) selon l'une quelconque des revendications précédentes, dans lequel l'unité d'éclairage (210) est positionnée sur un support (209).

8. Dispositif (240) selon l'une quelconque des revendications précédentes, comprenant un imageur (208).

9. Dispositif (240) selon l'une quelconque des revendications précédentes, comprenant une source d'alimentation (202).

10. Dispositif (240) selon l'une quelconque des revendications précédentes, comprenant un émetteur (212).

11. Dispositif (240) selon l'une quelconque des revendications précédentes, dans lequel ledit dispositif d'imagerie in vivo (240) est une capsule autonome.

12. Dispositif (240) selon l'une quelconque des revendications précédentes, dans lequel le champ de vision (223) se trouve dans le champ de vision proche, dans la plage de 0 à 5 cm à partir d'un hublot (221) du dispositif.
